Europäisches Patentamt
European Patent Office
Office européen des brevets

(19)

(11) Publication number: **0 320 501 A2**

# (12) EUROPEAN PATENT APPLICATION

(21) Application number: 89200049.8

(22) Date of filing: 25.06.84

(51) Int. Cl.⁴: **C 07 D 417/04**
C 07 D 277/24,
C 07 D 277/40,
C 07 D 277/42,
C 07 D 277/44,
C 07 D 417/12,
A 61 K 31/425, A 61 K 31/44

(30) Priority: 24.06.83 JP 113988/83
27.10.83 JP 201639/83
26.12.83 JP 248928/83

(43) Date of publication of application:
14.06.89 Bulletin 89/24

(84) Designated Contracting States:
AT BE CH DE FR GB IT LI LU NL SE

(60) Publication number of the earlier application in accordance with Art. 76 EPC: 0 130 077

(71) Applicant: YAMANOUCHI PHARMACEUTICAL CO. LTD.
No. 5-1 Nihonbashi-Honcho, 2-chome Chuo-ku
Tokyo (JP)

(72) Inventor: Kojima, Tadao
No. 1086-2, Oaza sanagaya Shiraoka-cho
Minamisaitama-gun Saitama (JP)

Kageyama, Shunji
No. 1-36-6, Nakano Nakano-ku
Tokyo (JP)

Okada, Minoru
No. 6-10-20, Higashioi Shinagawa-ku
Tokyo (JP)

Ohata, Isao
No. 47-17, Marugasaki-cho
Omiya-shi Saitama (JP)

(74) Representative: Geering, Keith Edwin et al
REDDIE & GROSE 16 Theobalds Road
London WC1X 8PL (GB)

## (54) Phenoxy derivatives, their preparation, and pharmaceutical compositions containing them.

(57) A phenoxy compound represented by general formula (I), or a salt thereof

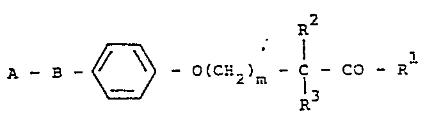 (I)

wherein:
A is an imidazolyl group, a pyridyl group, a $C_1$-$C_5$ alkyl group, an amino group which may be substituted with a phenyl group or a $C_1$-$C_5$ alkyl group, or a carbamoyl group substituted with a cyclohexylamino group or an imidazolylalkyl group;
B is a thiazolyl group which may be substituted with a $C_1$-$C_5$ alkyl group
m is 0 or an integer of 1 to 6;
$R^1$ is a hydroxyl group, an amino group or a $C_1$-$C_5$ alkoxy group; and
$R^2$ and $R^3$ are the same or different and selected from a hydrogen atom and $C_1$-$C_5$ alkyl groups.

The compounds are effective for prophylaxis and treatment of arteriosclerosis, cerebal infraction, transient ischemic attack, angina pectoris, peripheral thrombus and obstruction.

EP 0 320 501 A2

**Description**

## PHENOXY DERIVATIVES, THEIR PREPARATION, AND PHARMACEUTICAL COMPOSITIONS CONTAINING THEM

The present invention relates to novel phenoxy derivatives represented by general formula (I):

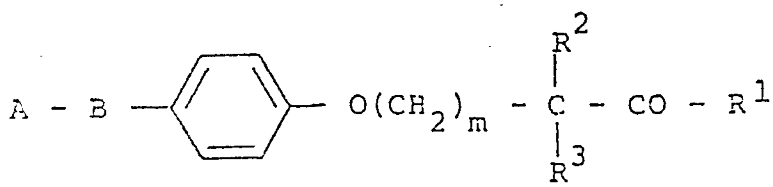

(I)

wherein:
A is an imidazolyl group, a pyridyl group, a lower alkyl group, an amino group which may be substituted with a phenyl group or a lower alkyl group, or a carbamoyl group substituted with a cyclohexylamino group or an imidazolylalkyl group;
B is a thiazolyl group which may be substituted with a lower alkyl group;
m is 0 or an integer of 1 to 6;
$R^1$ is a hydroxyl group, an amino group or a lower alkoxy group; and
$R^2$ and $R^3$ are the same or different and selected from a hydrogen atom and lower alkyl groups; and salts thereof.

In the definition of the groups appearing in the formulae in the specification, the term "lower" refers to a straight or branched carbon chain having 1 to 5 carbon atoms, preferably 1 to 3 carbon atoms. Accordingly, specific examples of lower alkyl groups include a methyl group, an ethyl group, a propyl group, an isopropyl group, a butyl group, an isobutyl group, a sec-butyl group, a tert-butyl group, a pentyl (or amyl) group, an isopentyl group, a neopentyl group, a tert-pentyl group, etc.

The compounds represented by general formula (I) described above form salts, which the present invention encompasses.

In particular, preferred examples of the salts include salts with inorganic acids such as hydrochloric acid, hydrobromic acid, phosphoric acid, etc.; salts with organic acids such as formic acid, acetic acid, lactic acid, oxalic acid, succinic acid, fumaric acid, benzoic acid, benzenesulfonic acid, etc. and quaternary ammonium salts with alkyl halides such as methyl iodide, etc.

Further the compounds of the present invention also include compounds in which geometric isomers and a variety of optical isomers (racemic isomers and optically active isomers, etc.) exist and the present invention covers all of these isomers.

The desired compounds of the present invention possess lipid lowering activity, particularly for reducing cholesterol and triglyceride, and also show activity in preventing platelet aggregation. The compounds of the present invention are effective for prophylaxis and treatment of arteriosclerosis, cerebral infraction, transient ischemic attack, angina pectoris, peripheral thrombus and obstruction.

Animal tests have shown that compounds of the present invention are active in reducing cholesterol and triglycerides and in selectively increasing high density lipoprotein (HDL) in blood. It is known that with arterioscrelosis the quantity of HDL decreases from the normal level, and that HDL prevents excessive accumulation of cholesterol in the arterial wall and accelerates the release of cholesterol from the arterial wall to the blood. In addition, compounds of the present invention show activity in preventing platelet aggregation induces by arachidonic acid. This activity combined with lipid reducing activity contributes to prophylaxis and treatment of the aforesaid various diseases such as arteriosclerosis, etc.

The pharmacological effects of compounds of the present invention have been confirmed as follows:

Lipid Reducing Activity:

Feed containing 1.5% cholesterol and 0.5% bile acid was given to male Sprague-Dawley rats aging 4 weeks old after birth for 7 days. A suspension of the compound of the present invention in a 0.5% aqueous methyl cellulose solution was administered daily via oral catheter for 4 days. After fasting overnight, blood was collected under ether anesthesia to determine the quantities of the total HDL and cholesterol in serum. The measurements of cholesterol and HDL were performed in accordance with the method described in Schettler, G. & Hüssel; Arbetsmed. Sozialmed. Präventived., 10, 25 (1975) and the method described in T.T. Ishikawa et al.; Lipids, 11, 628 (1976), respectively. The lipid reducing activity of representative compounds of the present invention is shown in Table I below.

Platelet Aggregation Prevention Activity:

Platelet rich plasma (PRP) and platelet poor plasma (PPP) were prepared from venous blood of Nippon white rabbit. The platelet aggregation activity was measured in accordance with the method described in Born, G. V. R.; Nature, 194, 927 (1962) and the activity of preventing platelet agglutination of the compound against platelet aggregation induced by arachidonic acid (final concentration, 0.2 mM) was determined with Aggrigometer (made by Payton Co.).

The platelet aggregation inhibitory activity of representative compounds of the present invention is shown in Table I.

Table I

| Structural Formula | Dose p.o. (mg/kg/day) | Hyperlipedemic rat: Reduction Rate of T* and Chol** (%) | HDL-Chol** cont.=1 | HDL-C/LDL-C cont.=1 | Platelet Aggregation inhibition (%) A.A. |
|---|---|---|---|---|---|
| Example 2: 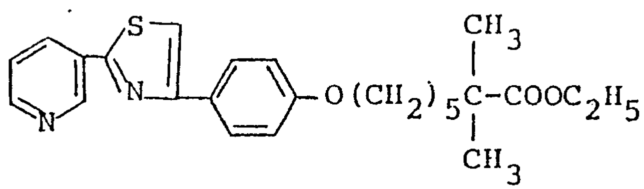 | 100 | 59 | 7.62 | 22 | 100 μM<br>16 |
| Example 5: 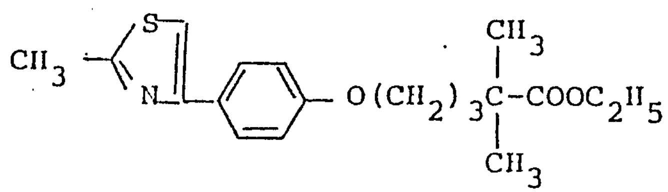 | 100 | 69 | 1.54 | 8.00 | 100 μM<br>50 |

Drugs containing the compounds represented by general formula (I) or salts thereof as active ingredients can be prepared using pharmaceutical carriers and vehicles conventionally used in the art in a conventional manner.

Drug administration may be oral e.g. in the form of powders, granules or capsules, or parenteral such as by injection, etc. but is preferably oral.

The appropriate dose depends upon the condition, age and sex, etc., of the patient; for example, in the case of oral administration, the dose may be in the range of 1 to 100 mg/kg, preferably 5 to 25 mg/kg, daily for an adult, given all at once or in 2 to 4 administrations.

According to the present invention, the compounds (I) of the present invention and salts thereof can be prepared by the method illustratively shown below.

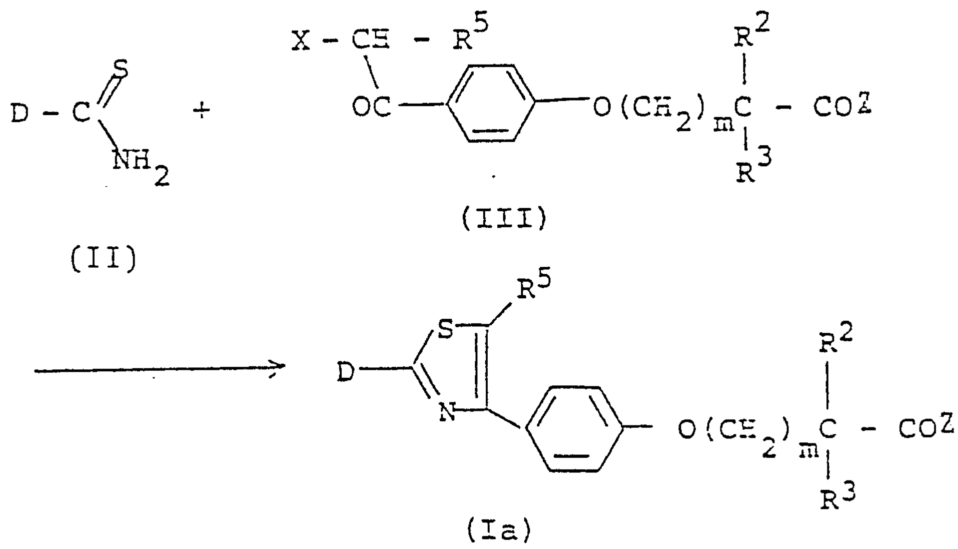

(II) (III) (Ia)

wherein Z represents an amino group or a group represented by formula -OR$^{1'}$ (wherein R$^{1'}$ is a hydrogen atom, a lower alkyl group or a protective group), D represents an imidazolyl group, a pyridyl group, a lower alkyl group, or an amino group which may be substituted with a phenyl group or a lower alkyl group; $R^5$ represents a hydrogen atom or a lower alkyl group; X represents a halogen atom and $R^2$, $R^3$ and m have the same significance as defined above.

To prepare the compounds of the present invention according to this method and thioamide compound represented by formula (II) and ω -(halogenoacetyloxy)alkyl carboxylic acids or esters thereof (III) are reacted in a solvent inert to the reaction.

Preferred examples of the solvents include acetone, and alcoholic solvents such as methanol, ethanol, etc. The proportion of compounds (II) to compounds (III) to be used is preferably almost equimolar.

The reaction easily proceeds at room temperature but if necessary or desired, heating is performed.

The thus obtained compounds can be converted to salts thereof by reacting with the aforesaid acids or bases. When Z is -OH the free acid product can be converted to ester in conventional manner.

When D is a free amino group, cycloalkyl isocyanates or imidazolylalkylcarboxylic acids can be reacted with the compounds represented by formula (Ia). The reaction with cycloalkyl isocyanates can be conducted under reflux using tetrahydrofuran, etc. as a solvent. The reaction with imidazolylalkylcarboxylic acids can be performed with heating in N,N-dimethylformamide (hereafter referred to as DMF) in the presence of dicyclohexylcarbodiimide (hereafter referred to as DCC).

Examples of the protective group used are an optionally substituted benzyl group, a trityl group, an alkyloxyalkyl group, a phenacyl group, etc.

Further, when R$^{1'}$ in formulae Ia is a lower alkyl group or a protective group, the group may optionally be removed in conventional manner.

In some preferred compounds according to the invention, A is a $C_1$-$C_5$ alkyl group or a pyridyl group, E is a thiazolyl group, $R^1$ is a $C_1$-$C_5$ alkoxy group and $R^2$, $R^3$ and $R^4$ are $C_1$-$C_5$ alkyl groups.

The compounds of the present invention prepared as described above may be isolated as they are or as the salts and purified. Isolation and purification can be performed in conventional manner such as crystallization, distillation, extraction, various chromatography techniques, recrystallization, etc.

Hereafter the present invention will be described in more detail with reference to the Examples. Some of the starting compounds of the present invention are novel and preparation of such compounds is shown in the Reference Examples below.

REFERENCE EXAMPLE 1

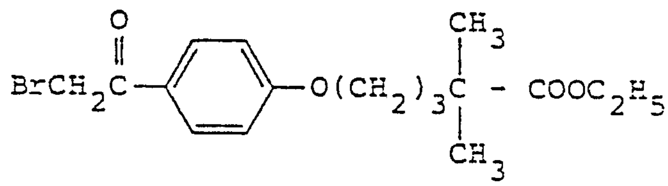

A mixture of 30 g of p-hydroxyacetophenone and 30 g of potassium carbonate was stirred in 500 ml of ethanol at 60°C for 30 minutes. After cooling to room temperature, 52 g of ethyl 5-bromo-2,2-dimethylpentanoate was added to the reaction mixture. The resulting mixture was heated under reflux overnight while stirring. The solvent was removed from the reaction mixture by distillation and 500 ml of chloroform and 300 ml of a 5% aqueous hydrogen sodium carbonate solution were added to the residue. After stirring the mixture, the chloroform layer was fractionated and washed successively with water and then with a saturated aqueous sodium chloride solution followed by drying over anhydrous sodium sulfate. Removal of chloroform by distillation gave a residual oily substance. The oily substance was subjected to silica gel column chromatography and eluted using chloroform as an eluant to give 42 g of ethyl p-acetylphenoxy-2,2-dimethylpentanoate as an oily substance. The thus obtained oily substance was dissolved in 1.6 l. of anhydrous methylene chloride and 24 g of bromine was dropwise added to the solution under ice cooling while stirring, with reaction for 3 hours. To the reaction mixture was added 1 l. of ice water. After thoroughly stirring, the organic layer was successively washed with water and with a saturated aqueous sodium chloride solution followed by drying over anhydrous sodium sulfate. Removal of methylene chloride by distillation gave the product ethyl 5-[p-(bromoacetyl)phenoxy]-2,2-dimethylpentanoate, as an oily substance.

REFERENCE EXAMPLE 2

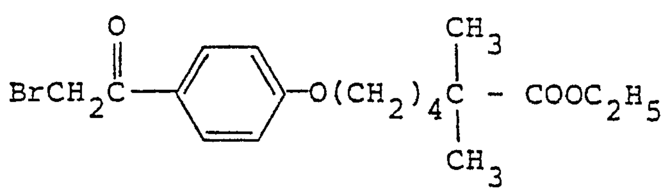

Ethyl 6-bromo-2,2-dimethylhexanoate was used as the starting material in place of ethyl 5-bromo-2,2-dimethylpentanoate in REFERENCE EXAMPLE 1. The starting material was reacted and treated in a manner similar to REFERENCE EXAMPLE 1 to give the product ethyl 6-[p-bromoacetylphenoxy]-2,2-dimethylhexanoate as an oily substance.

REFERENCE EXAMPLE 3

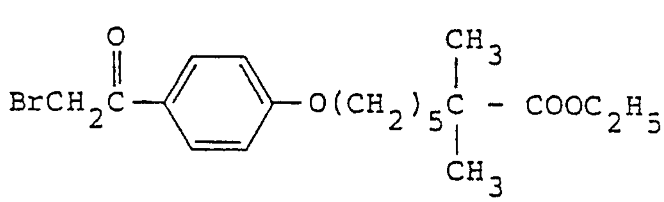

Ethyl 7-bromo-2,2-dimethylheptanoate was used as the starting material in place of ethyl 5-bromo-2,2-dimethylpentanoate in REFERENCE EXAMPLE 1. The starting material was reacted and treated in a manner similar to REFERENCE EXAMPLE 1 to give the product ethyl 7-[p-bromoacetylphenoxy]-2,2-dimethylheptanoate as an oily substance.

## REFERENCE EXAMPLE 4

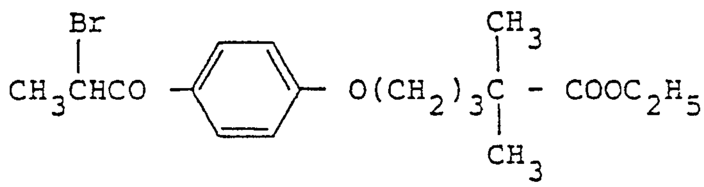

p-Hydroxypropiophenone was used as the starting material in place of p-hydroxyacetophenone in REFERENCE EXAMPLE 1. The starting material was reacted and treated in a manner similar to REFERENCE EXAMPLE 1 to give the product ethyl 5-[p-(2-bromopropionyl)-phenoxy]-2,2-dimethylpentanoate as an oily substance.

## REFERENCE EXAMPLE 5

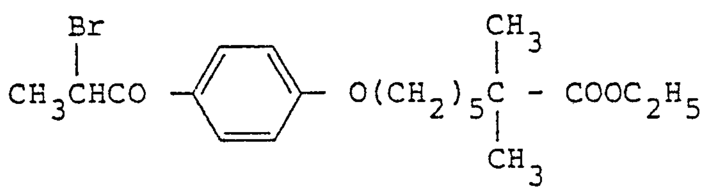

p-Hydroxypropionphenone was used as the starting material in place of p-hydroxacetophenone in REFERENCE EXAMPLE 3. The starting material was reacted and treated in a manner similar to REFERENCE EXAMPLE 3 to give the product ethyl 7-[p-(2-bromopropionyl)phenoxy]-2,2-dimethylheptanoate as an oily substance.

## EXAMPLE 1

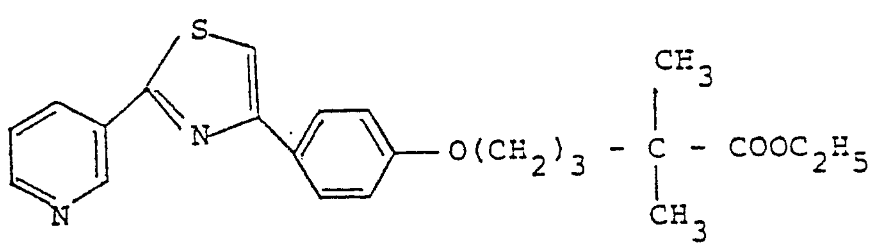

Ethyl 5-[p-(2-bromoacetyl)phenoxy]-2,2-dimethylpentanoate, 1.8 g, obtained in REFERENCE EXAMPLE 1

and 0.7 g of 3-pyridylthioamide were stirred in 20 ml of methanol at room temperture for 5 hours. The solvent was removed from the reaction mixture by distillation and, 30 ml of chloroform and 30 ml of a 5% aqueous hydrogen sodium carbonate solution were added to the residue. After the mixture was stirred, the chloroform layer was fractionated and successively washed with water and an aqueous saturated sodium chloride solution followed by drying over anhydrous sodium sulfate. Chloroform was distilled off to give the oily residue.

The oily residue was subjected to silica gel column chromatography and the product was eluted out using chloroform-methanol (10 : 1 in a volume ratio) as an eluant. The solvent was removed from the elute by distillation under reduced pressure to obtain the product ethyl 2,2-dimethyl-5-[p-[2-(3-pyridyl)-4-thiazolyl]phenoxy]pentanoate, as an oily substance.

NMR Spectrum ($CDCl_3$) (internal standard: TMS)

δ (ppm):

1.22 (6H, s, $CH_3$-C-$CH_3$)

1.26 (3H, t, J=7.2Hz $COOCH_2$-$CH_3$)

1.5 - 1.9 (4H, m, C-$CH_2$-$CH_2$-C)

3.99 (2H, t, J=5.4Hz, Ø-O-$CH_2$-C)

4.12 (2H, q, J=7.2Hz, $COOCH_2$-C)

6.94 (2H, d, J=8.3Hz, 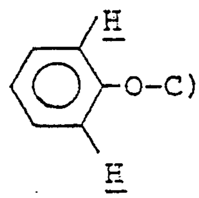

7.38 (1H, s 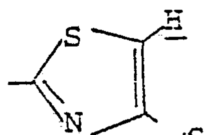 )

7.38 (1H, m, 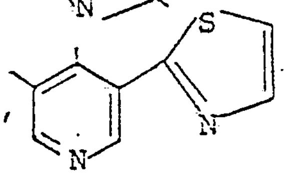 )

8.30 (1H, d,t, J=7.2Hz, J=1.8Hz,

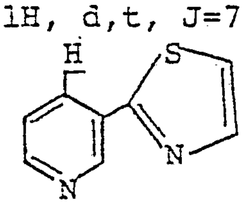 )

8.64 (1H, dd, J=4.3Hz, J=1.8Hz,

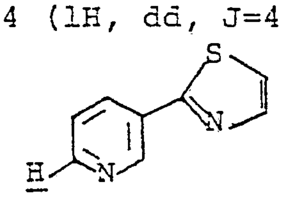 )

7.90 (2H, d, J=8.3Hz, 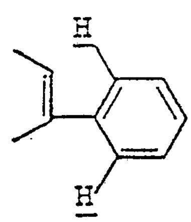 )

8.20 (1H, d, J=1.8Hz,

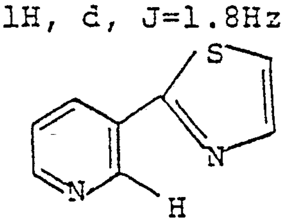 )

EXAMPLE 2

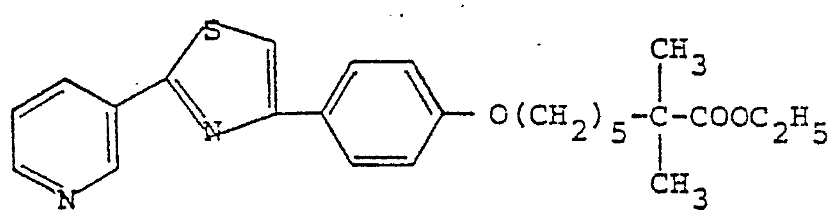

Ethyl 7-[p-(bromoacetyl)phenoxy]-2,2-dimethylheptanoate obtained in REFERENCE EXAMPLE 3 was used as the starting material in place of ethyl 5-[p-(2-bromoacetyl)phenoxy]-2,2-dimethylpentanoate in EXAMPLE 1. The starting material was reacted and treated in a manner similar to EXAMPLE 1. The resulting residue was recrystallized from petroleum ether to give the product ethyl 2,2-dimethyl-7-[p-[2-(3-pyridyl)-4-thiazolyl]phenoxy]heptanoate, as white crystals.
Melting point: 41 - 42°C

Elemental Analysis (as $C_{25}H_{30}N_2O_3S$)

| | C(%) | H(%) | (N%) | (S%) |
|---|---|---|---|---|
| Calcd. | 68.46 | 6.89 | 6.39 | 7.31 |
| Found | 68.60 | 6.87 | 6.36 | 7.11 |

EXAMPLE 3

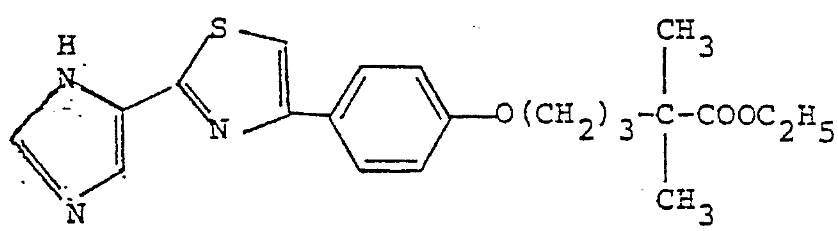

After 3.6 g of ethyl 5-[p-(bromoacetyl)phenoxy]-2,2-dimethylpentanoate obtained in REFERENCE EXAMPLE 1 and 1 g of 4-imidazolylthioamide were stirred in 40 ml of methanol at room temperature for 15 minutes, the mixture was stirred with heating under reflux at 70°C for 2 hours. Then, the solvent was removed from the reaction mixture by distillation and 200 ml of chloroform and 100 ml of a 5% aqueous hydrogen sodium carbonate solution were added to the residue. After strring the mixture, the chloroform layer was fractionated and successively washed with water and then an aqueous saturated sodium chloride solution followed by drying over anhydrous sodium sulfate. Chloroform was removed by distillation to give an oily substance. The oily substance was subjected to silica gel column chromatography. The product was eluted out using chloroform-methanol (20:1 in a volume ratio) as an eluant. The solvent was removed from the elute by distillation under reduced pressure. The residue was recrystallized from ethyl acetate to obtain the product ethyl 5-[p-[2-(4-imidazolyl)-4-thiazolyl]phenoxy]-2,2-dimethylpentanoate as white crystals.
Melting point: 122 - 123°C

Elemental Analysis (as $C_{21}H_{25}N_3O_3S$)

| | C(%) | H(%) | (N%) | (S%) |
|---|---|---|---|---|
| Calcd. | 63.14 | 6.31 | 10.52 | 8.02 |
| Found | 63.21 | 6.25 | 10.46 | 8.14 |

EXAMPLE 4

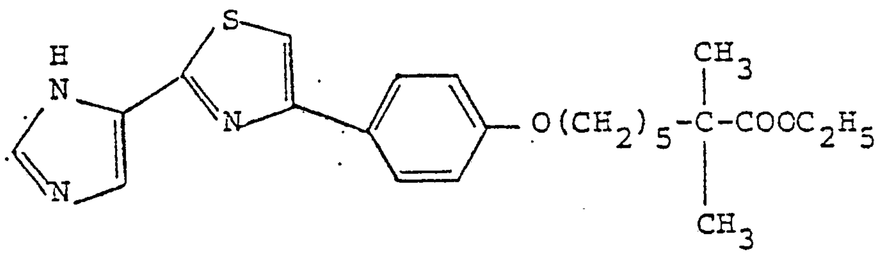

Ethyl 7-[p-(bromoacetyl)phenoxy]-2,2-dimethylheptanoate obtained in REFERENCE EXAMPLE 3 was used as the starting compound in place of ethyl 5-[p-(bromoacetyl)phenoxy]-2,2-dimethylpentanoate in EXAMPLE 3. The starting compound was reacted and treated in a manner similar to EXAMPLE 3 (except that recrystallization was performed from ethyl acetate-hexane) to obtain the product ethyl 5-[p-[2-(4--imidazolyl)-4-thiazolyl]phenoxy]-2,2-dimethylheptanoate as white crystals.
Melting point: 114 - 115°C

Elemental Analysis (as $C_{23}H_{29}N_3O_3S$)

| | C(%) | H(%) | (N%) | (S%) |
|---|---|---|---|---|
| Calcd. | 64.61 | 6.84 | 9.83 | 7.50 |
| Found | 64.69 | . 7.02 | 9.75 | 7.40 |

EXAMPLE 5

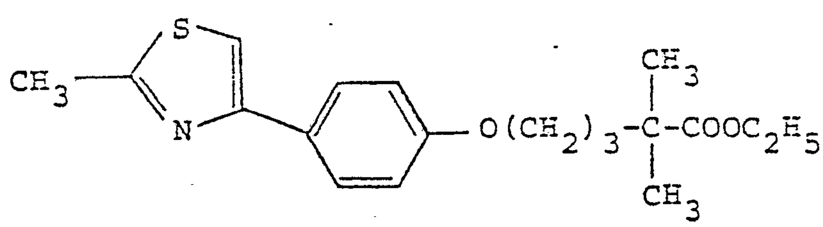

Thioacetamide was used as the starting compound in place of 4-imidazolylthioamide in EXAMPLE 3. The starting compound was reacted and treated in a manner similar to EXAMPLE 3. The resulting oily substance was subjected to silica gel column chromatography. The product was eluted out using chloroform as an eluant and the solvent was removed from the elute by distillation under reduced pressure to obtain the product ethyl 5-[p-(2-methyl-4-thiazolyl)phenoxy]-2,2-dimethylpentanoate as an oily substance.
NMR Spectrum ($CDCl_3$) (internal standard: TMS)

δ (ppm):

1.20 (6H, s, $CH_3$-C-$CH_3$ )

1.22 (3H, t, J=7.2Hz, COOC-$CH_3$)

1.5-1.9 (4H, m, C-$CH_2$-$CH_2$-C)

2.73 (3H, s, 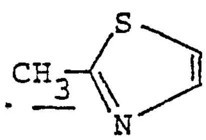 )

3.94 (2H, t, J=6.1Hz, Ø-O-$CH_2$-C)

4.08 (2H, q, J=7.2Hz, COO-$CH_2$-C)

6.86 (2H, d, J=8.3Hz, 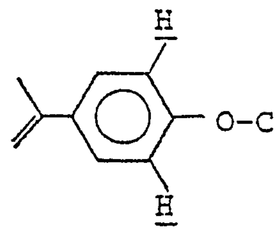)

7.12 (1H, s, 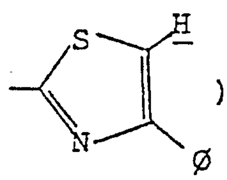 )

7.74 (2H, d, J=8.3Hz, 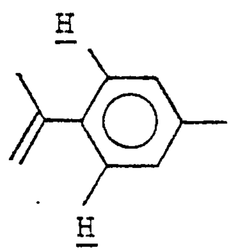 )

EXAMPLE 6

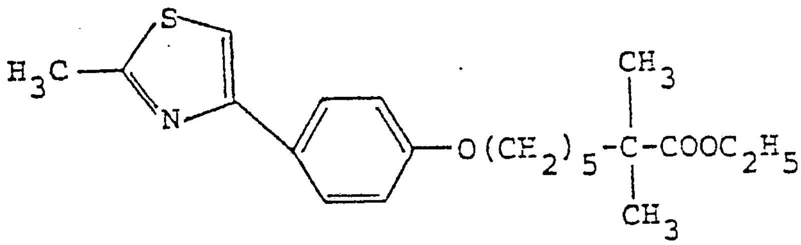

Thioacetamide was used as the starting compound in place of 3-pyridylacetamide in EXAMPLE 2. The starting compound was reacted and treated in a manner similar to EXAMPLE 1. The resulting oily substance was subjected to silica gel column chromatography. The product was eluted out using n-hexane-ethyl acetate (8:1 in a volume ratio) as an eluant and the solvent was removed from the elute by distillation under reduced pressure to give the product ethyl 7-[p-(2-methyl-4-thiazolyl)phenoxy]-2,2-dimethylheptanoate as an oily substance.

NMR Spectrum ($CDCl_3$) (internal standard: TMS)

δ (ppm):

1.16 (6H, s, $\underline{CH_3}$-C-$\underline{CH_3}$)

1.23 (3H, t, J=7.2Hz, $\underline{CH_3}$-$CH_2$-O-)

1.1-1.9 (8H, m, 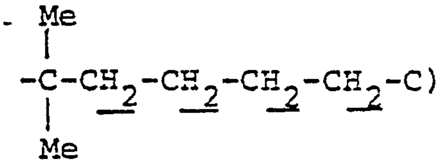

2.76 (3H, s, 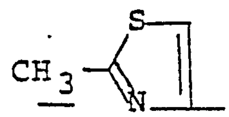 )

3.98 (2H, t, J=6.1Hz, 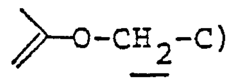

4.12 (2H, q, J=7.2Hz, $COO-CH_2-C$)

6.92 (2H, d, J=9.0Hz, 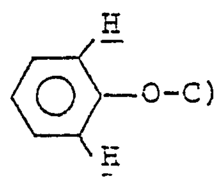

7.16 (1H, s, 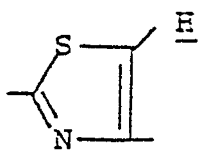 )

7.80 (1H, d, J=9.0Hz, 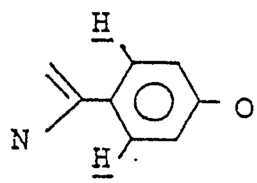 )

EXAMPLES 7 TO 9

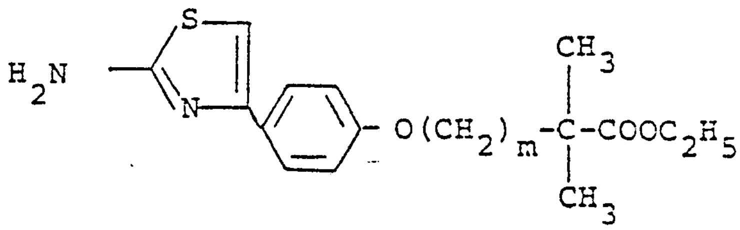

(m = 3 to 5)

The ethyl bromoacetylphenoxycarboxylates obtained in REFERENCE EXAMPLES 1 to 3 were used as the starting compounds in combination with thiourea. The starting compounds were reacted and treated in a manner similar to EXAMPLE 1 to prepare the compounds of EXAMPLES 7 to 9.

| EXAMPLE No. | Starting Compound REFERENCE EXAMPLE No. | Product: Structural Formula | Product: Melting Point (°C) | Elemental Analysis: C(%) | H(%) | N(%) | S(%) |
|---|---|---|---|---|---|---|---|
| 7 | 1 | 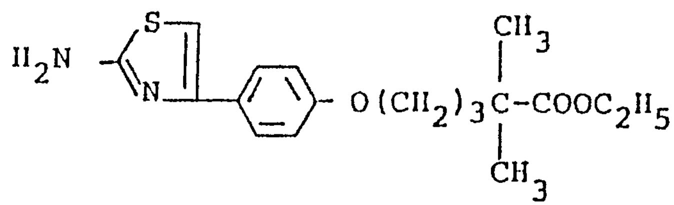 Ethyl 5-[p-(2-amino-4-thiazolyl)phenoxy]-2,2-dimethylpentanoate | 127-128 | (as $C_{18}H_{24}N_2O_3S$) | | | |
| | | | | Calcd. | | | |
| | | | | 62.04 | 6.94 | 8.04 | 9.20 |
| | | | | Found | | | |
| | | | | 61.88 | 7.05 | 7.86 | 9.01 |
| 8 | 2 | 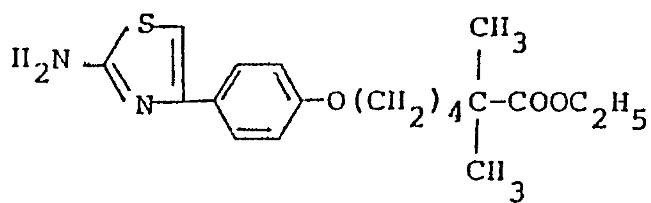 Ethyl 6-[p-(2-amino-4-thiazolyl)-phenoxy]-2,2-dimethylhexanoate | 138-140 | (as $C_{19}H_{26}N_2O_3S$) | | | |
| | | | | Calcd. | | | |
| | | | | 62.96 | 7.23 | 7.73 | 8.84 |
| | | | | Found | | | |
| | | | | 62.81 | 7.31 | 7.55 | 9.11 |

| EXAMPLE No. | Starting Compound REFERENCE EXAMPLE No. | Product: Structural Formula | Product: Melting Point (°C) | Product: Elemental Analysis C(%) | H(%) | N(%) | S(%) |
|---|---|---|---|---|---|---|---|
| 9 | 3 | 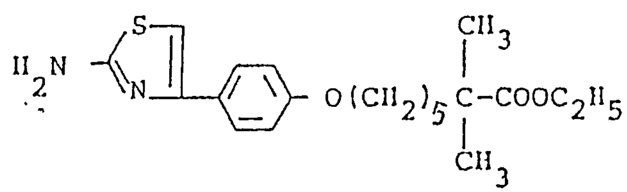 Ethyl 7-[p-(2-amino-4-thiazolyl)-phenoxy]-2,2-dimethylheptanoate | 117-119 | (as $C_{20}H_{28}N_2O_3S$) Calcd. 63.80 | 7.50 | 7.44 | 8.51 |
| | | | | Found 63.77 | 7.54 | 7.34 | 8.50 |

EXAMPLES 10 AND 11

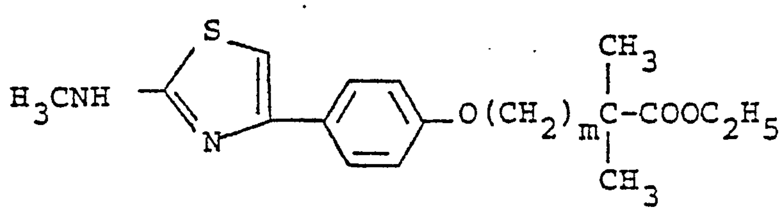

(m = 3, 4)

The ethyl bromoacetylphenoxycarboxylates obtained in REFERENCE EXAMPLES 1 and 2 were used as the starting compounds in combination with methyl thiourea. The starting compounds were reacted and treated in a manner similar to EXAMPLE 1 to prepare the compounds of EXAMPLES 10 and 11.

| EXAMPLE No. | Starting Compound REFERENCE EXAMPLE No. | Product: Structural Formula | Product: Melting Point (°C) | Product: Elemental Analysis C(%) H(%) N(%) S(%) |
|---|---|---|---|---|
| 10 | 1 | 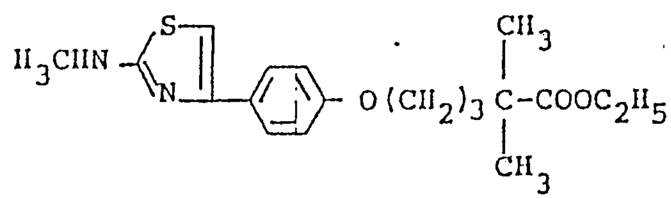<br>Ethyl 2,2-dimethyl-5-[p-(2-methylamino-4-thiazolyl)phenoxy]-pentanoate | 65-66 | (as $C_{19}H_{26}N_2O_3S$)<br>Calcd.<br>62.96 7.23 7.73 8.84<br>Found<br>62.96 7.46 7.63 8.74 |
| 11 | 2 | 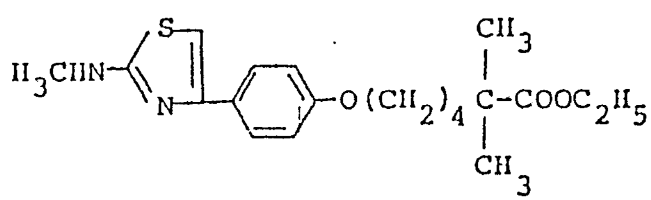<br>Ethyl 2,2-dimethyl-6-[p-(2-methylamino-4-thiazolyl)-phenoxy]hexanoate | 93-95 | (as $C_{20}H_{28}N_2O_3S$)<br>Calcd.<br>63.80 7.50 7.44 8.51<br>Found<br>63.92 7.57 7.43 8.54 |

EXAMPLES 12 AND 13

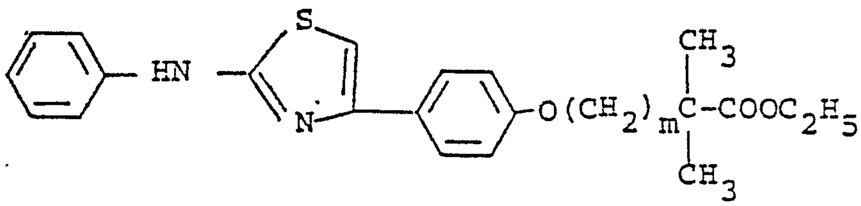

(m = 3, 4)

The ethyl bromoacetylphenoxycarboxylates obtained in REFERENCE EXAMPLES 1 and 2 were used as the starting compounds in combination with 1-phenyl-2-thiourea. The starting compounds were reacted and treated in a manner similar to EXAMPLE 1 to prepare the compounds of EXAMPLES 12 and 13.

| EXAMPLE No. | Starting Compound REFERENCE EXAMPLE No. | Product: Structural Formula | Product: Melting Point (°C) | Elemental Analysis: C(%) | H(%) | N(%) | S(%) |
|---|---|---|---|---|---|---|---|
| 12 | 1 | 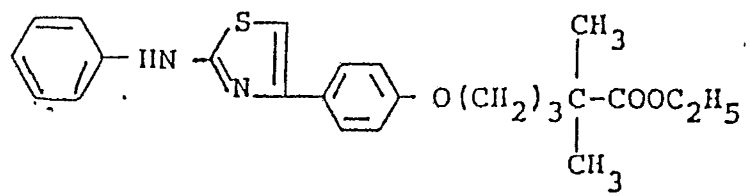 | 85-86 | (as $C_{24}H_{28}N_2O_3S$) | | | |
| | | | | Calcd. | | | |
| | | | | 67.90 | 6.65 | 6.60 | 7.75 |
| | | | | Found | | | |
| | | Ethyl 2,2-dimethyl-5-[p-(2-phenylamino-4-thiazolyl)-phenoxy]pentanoate | | 68.00 | 6.72 | 6.35 | 7.30 |
| 13 | 2 | 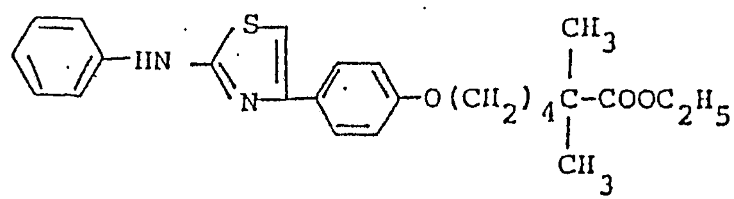 | 65-68 | (as $C_{25}H_{30}N_2O_3S$) | | | |
| | | | | Calcd. | | | |
| | | | | 68.46 | 6.89 | 6.39 | 7.31 |
| | | | | Found | | | |
| | | Ethyl 2,2-dimethyl-6-[p-(2-phenylamino-4-thiazolyl)-phenoxy]hexanoate | | 68.60 | 7.03 | 6.29 | 7.11 |

## EXAMPLES 14 TO 16

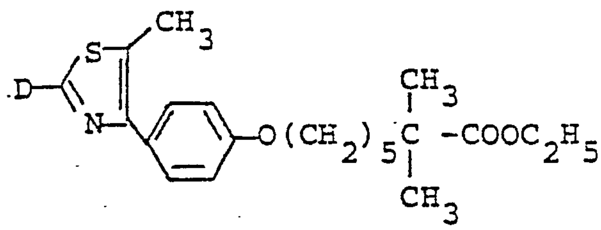

( D = 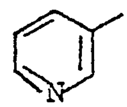 , $-CH_3$ , $-NH_2$ )

Ethyl 7-[p-(2-bromopropionyl)phenoxy]-2,2-dimethylheptanoate obtained in REFERENCE EXAMPLE 5 was used in combination with 3-pyridylthioamide, thioacetamide and thiourea, respectively, as the starting compounds. The starting compounds were reacted and treated in a manner similar to EXAMPLE 3 to prepare the compounds of EXAMPLES 14 to 16.

| EXAMPLE No. | Starting Compound | Product: Structural Formula | Product: Melting Point (°C) | Product: Elemental Analysis NMR Spectrum |
|---|---|---|---|---|
| 14 | 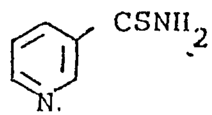 | 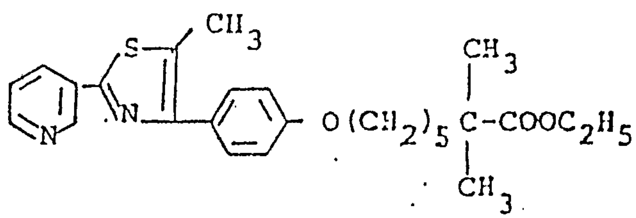<br>Ethyl 2,2-dimethyl-7-[p-[5-methyl-2-(3-pyridyl)-4-thaizolyl]phenoxy]heptanoate | oily substance | NMR Spectrum ($CDCl_3$)<br>(internal standard: TMS)<br>δ (ppm):<br>1.16(6H,s, $\underline{CH_3}$-C-$\underline{CH_3}$)<br>1.23(3H, t, J=7.2Hz, COO-C-$\underline{CH_3}$)<br>1.1-1.9 (8H, m, C-$\underline{CH_2CH_2}$-$\underline{CH_2CH_2}$-C)<br>2.62 (3H,s, 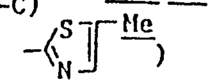)<br>4.02(2H,t, 6.1Hz, ≻O-$\underline{CH_2}$-C)<br>4.12 (2H,q, J=7.2Hz, COO$\underline{CH_2}$-C)<br>6.98 (2H, d, J=9Hz, 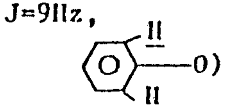) |

| EXAMPLE No. | Starting Compound | Product | | |
|---|---|---|---|---|
| | | Structural Formula | Melting Point (°C) | Elemental Analysis<br>NMR Spectrum |
| | | | | NMR spectrum ($CDCl_3$)<br>(internal standard: TMS)<br>$\delta$ (ppm):<br>7.34 (1H, dd, J=8.3Hz, J=4.3Hz 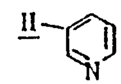 )<br>7.66 (2H, d, J=9Hz, 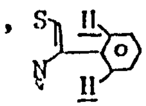 )<br>8.26 (1H,d,t, J=8.3Hz, J=1.8Hz, 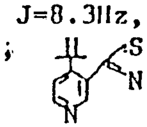 )<br>8.64 (1H,d,d, J=4.3Hz, J=1.8Hz, 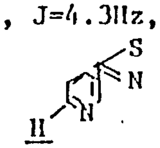 )<br>9.16 (1H, d, J=1.8Hz, 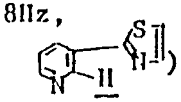) |

| EXAMPLE No. | Starting Compound | Product | | Elemental Analysis |
|---|---|---|---|---|
| | | Structural Formula | Melting Point (°C) | NMR Spectrum |
| 15 | $CH_3CSNH_2$ | 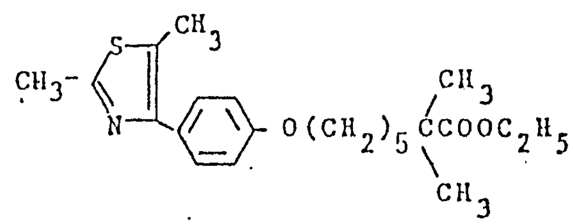<br>Ethyl 2,2-dimethyl-7-[p-2,5-dimethyl-4-thiazolyl)-phenoxy]heptanoate | oily substance | NMR spectrum ($CDCl_3$)<br>(internal standard: TMS)<br>δ (ppm):<br>1.16(6H, s, $CH_3-C-CH_3$)<br>1.23(3H, t, J=7.2Hz, $CH_3-CH_2-O-$)<br>1.1~1.9(8H, m, $-C-CH_2-CH_2-CH_2-CH_2-C$)<br>2.46(3H, s, 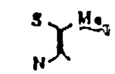 )<br>2.64(3H, s, Me-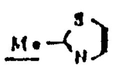 )<br>3.98(2H, t, J=5.8Hz, 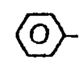$-O-CH_2-C$)<br>4.12(2H, q, J=7.2Hz, $COOCH_2-C$) |

| EXAMPLE No. | Starting Compound | Product: Structural Formula | Product: Melting Point (°C) | Product: Elemental Analysis / NMR Spectrum |
|---|---|---|---|---|
| 16 | $NH_2CSNH_2$ | 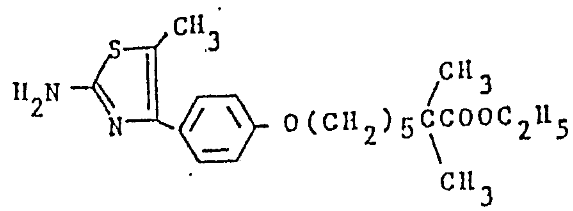 Ethyl 7-[P-(2-amino-5-methyl-4-thiazolyl)phenoxy]-2,2-dimethyl heptanoate | 93-94 | NMR spectrum ($CDCl_3$) (internal standard: TMS) δ (ppm): 6.93(2H, d, J=9.0Hz, 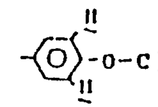 ) 7.54(2H, d, J=9.0Hz, 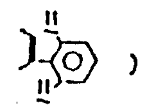 ) (as $C_{12}H_{30}N_2O_3S$) C(%) H(%) N(%) S(%) Calcd. 64.59 7.74 7.17 8.21 Found 64.58 7.85 7.05 8.10 |

EXAMPLE 17

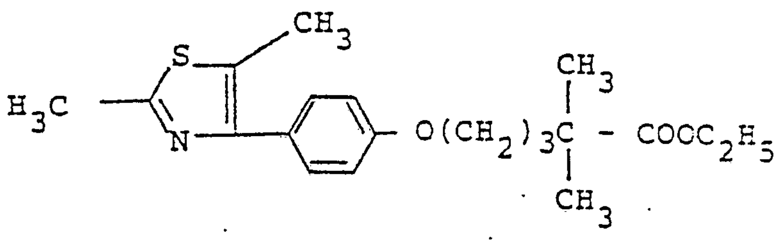

Ethyl 2,2-dimethyl-5-[p-(2-bromopropionyl)phenoxy]pentanoate obtained in REFERENCE EXAMPLE 4 was used as the starting compound in place of ethyl 7-[p-(2-bromopropionyl)phenoxy]-2,2-dimethylheptanoate in EXAMPLE 15. The starting compound was reacted and treated in a manner similar to EXAMPLE 15. The resulting oily substance was subjected to silica gel column chromatography. The solvent was removed from the elute by distillation under reduced pressure to give the product ethyl 5-[p-[2,5-dimethyl-4-thiazolyl)-4-thiazolyl]phenoxy]-2,2-dimethylpentanoate as an oily substance.
NMR Spectrum ($CDCl_3$) (internal standard: TMS)

δ (ppm) :

1.20 (6H, s, $CH_3$-C-$CH_3$ )

1.22 (3H, t, J=7.2Hz, -COOC-$CH_3$)

1.5-1.8 (4H, m, C-$CH_2$$CH_2$-C- )

2.48 (3H, s, 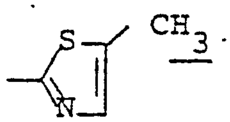 )

2.65 (3H, s, 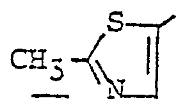 )

3.98 (2H, s, Ø-O$CH_2$-C )

4.12 (2H, q, J=7.2Hz, COO$CH_2$C )

6.91 (2H, d, J=8.3Hz, 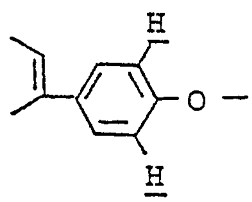

7.52 (2H, d, J=8.3Hz,

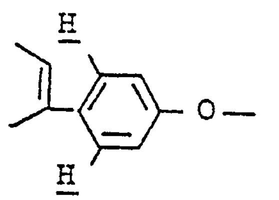

EXAMPLE 18

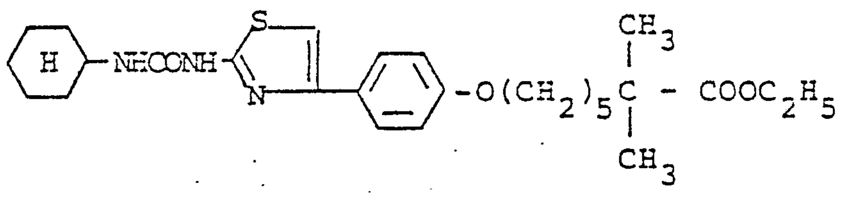

In 15 ml of tetrahydrofuran, 1.13 g of ethyl 7-[p-(2-amino-4-thiazolyl)phenoxy]-2,2-dimethyl-heptanoate obtained in EXAMPLE 9 was reacted with 0.38 g of cyclohexyl isocyanate overnight with heating under reflux. Ice water was poured into the mixture which was then extracted with methylene chloride. After the methylene chloride layer was dried over anhydrous magnesium sulfate, the solvent was removed under reduced pressure.The resulting residue was subjected to silica gel column chromatography. The product was eluted out using n-hexane-ethyl acetate (4:1 in the volume ratio) as an eluant. After the solvent was removed in vaccuo, the resulting crystals were recrystallized from a solvent mixture of n-hexane-ether to give ethyl 7-[p-[2-(3-cyclohexylureido)-4-thiazolyl]phenoxy]-2,2-dimethylheptanoate as white crystals.
Melting point: 106 - 108°C

Elemental Analysis (as $C_{27}H_{39}N_3O_4S$)

| | C(%) | H(%) | (N%) | (S%) |
|---|---|---|---|---|
| Calcd. | 64.64 | 7.84 | 8.38 | 6.39 |
| Found | 64.43 | 8.04 | 8.24 | 6.40 |

EXAMPLE 19

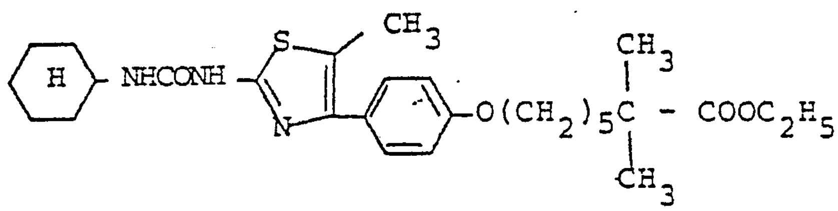

Ethyl 7-[p-(2-amino-5-methyl-4-thiazolyl)phenoxy]-2,2-dimethylheptanoate obtained in EXAMPLE 16 was used as a starting compound in place of ethyl 7-[p-(2-amino-4-thiazolyl)phenoxy]-2,2-dimethylheptanoate IN EXAMPLE 18. The starting compound was reacted and treated in a manner similar to EXAMPLE 18 to give the product ethyl 7-[p-[2-(3-cyclohexylureido)-5-methyl-4-thiazolyl]phenoxy]-2,2-dimethylheptanoate as white crystals.
Melting point: 79 - 81°C

Elemental Analysis (as $C_{28}H_{41}N_3O_4S$)

| | C(%) | H(%) | (N%) | (S%) |
|---|---|---|---|---|
| Calcd. | 65.21 | 8.01 | 8.15 | 6.22 |
| Found | 65.33 | 8.22 | 8.03 | 6.20 |

EXAMPLE 20

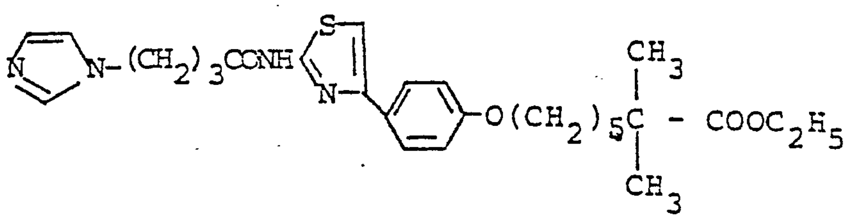

In 15 ml of dry N,N-dimethylformamide (DMF) were dissolved 0.39 g of 4-(1-imidazolyl )butyric acid and 0.37 g of 1-hydroxybenzotriazole. While stirring, 0.57 g of dicyclohexylcarbodiimide was added to the solution at 40 to 45°C. After dicyclohexylurea was precipitated out in the reaction mixture, a solution of 0.94 g of ethyl 7-[p-(2-amino-4-thiazolyl)phenoxy]-2,2-dimethyl heptanoate in 5 ml of DMF was dropwise added to the mixture at the same temperature and the mixture was stirred at the same temperature overnight. After removing dicyclohexylurea by filtration, the filtrate was condensed under reduced pressure. Ice water was added to the residue followed by extracting with chloroform. After the chloroform layer was washed successively with a saturated aqueous hydrogen sodium carbonate and water, the chloroform layer was dried over anhydrous sodium magnesium. The chloroform was removed by distillation under reduced pressure and the resulting residue was subjected to silica gel column chromatography. The product was eluted out using chloroform-methanol (40:1 in a volume ratio) as an eluant. After the solvent was removed by distillation, the resulting crystals were recrystallized from a solvent mixture of ether-benzene to give ethyl 7-[p-[2-[4-(1-imidazolyl)butylamido]-4-thiazolyl]phenoxy]-2,2-dimethylheptanoate as white crystals.
Melting point: 148 - 150°C

Elemental Analysis (as $C_{27}H_{36}N_4O_4S$)

| | C(%) | H(%) | (N%) | (S%) |
|---|---|---|---|---|
| Calcd. | 63.26 | 7.08 | 10.93 | 6.25 |
| Found | 63.01 | 7.08 | 10.85 | 6.13 |

EXAMPLE 21

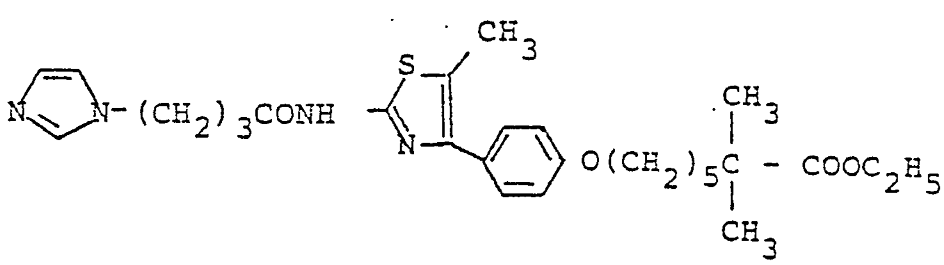

Ethyl 7-[p-(2-amino-5-methyl-4-thiazolyl)phenoxy]-2,2-dimethylheptanoate obtained in EXAMPLE 16 was used as a starting compound in place of ethyl 7-[p-(2-amino-4-thiazolyl)phenoxy]-2,2-dimethylheptanoate in EXAMPLE 20. The starting material was reacted and treated in a manner similar to EXAMPLE 20 to give the product ethyl 7-[p-[2-[4-(1-imidazolyl)butyramido]-5-methyl-4-thiazolyl]phenoxy]-2,2-dimethylheptanoate as white crystals.
Melting point: 85 - 87°C

Elemental Analysis (as $C_{28}H_{38}N_4O_4S$)

| | C(%) | H(%) | (N%) | (S%) |
|---|---|---|---|---|
| Calcd. | 63.85 | 7.27 | 10.64 | 6.09 |
| Found | 63.87 | 7.47 | 10.62 | 6.25 |

EXAMPLE 22

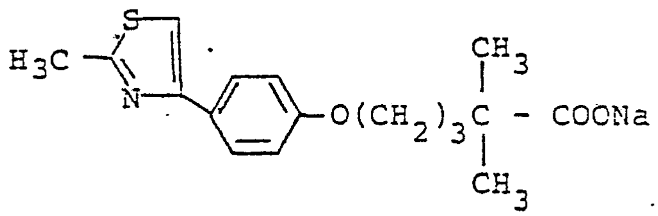

In 5 ml of ethanol was dissolved 1.40 g of ethyl 2,2-dimethyl-5-[p-(2-methyl-4-thiazolyl)phenoxy]pentanoate obtained in EXAMPLE 5. The solution was added to an aqueous sodium hydroxide solution (0.4 g of sodium hydroxide in 5 ml of water) and the mixture refluxed for 10 hours. After cooling, the precipitated crystals were washed with water to obtain the product sodium 2,2-dimethyl-5-[p-(2-methyl-4-thiazolyl)phenoxy]pentanoate as white crystals.
Melting point: 259 - 261°C

Elemental Analysis (as $C_{17}H_{20}NO_3SNa$)

| | C(%) | H(%) | N(%) | S(%) |
|---|---|---|---|---|
| Calcd. | 59.81 | 5.90 | 4.10 | 9.39 |
| Found | 58.72 | 5.73 | 4.12 | 9.34 |

EXAMPLE 23

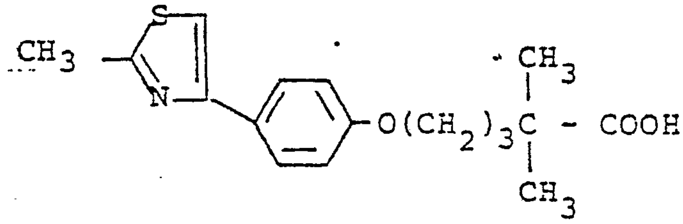

In 50 ml of water was dissolved 1.0 g of sodium 2,2-dimethyl-5-[p-(2-methyl-4-thiazolyl)phenoxy]pentanoate obtained in EXAMPLE 22. The solution was neutralized with 10% hydrochloric acid. The precipitated crystals were taken out by filtration and washed with water to give the product 2,2-dimethyl-5-[p-(2-methyl-4-thiazolyl)phenoxy]pentanoic acid as white crystals.
Melting point: 150 - 153°C

Elemental Analysis (as $C_{17}H_{21}NO_3S$)

| | C(%) | H(%) | (N%) | (S%) |
|---|---|---|---|---|
| Calcd. | 63.92 | 6.63 | 4.39 | 10.04 |
| Found | 63.67 | 6.65 | 4.13 | 9.64 |

EXAMPLE 24

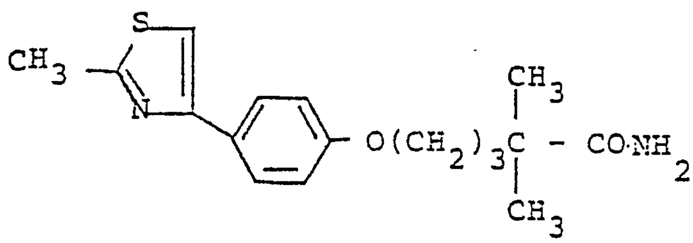

In 10 ml of dichloromethane were dissolved 0.7 g of 2,2-dimethyl-5-[p-(2-methyl-4-thiazolyl)phenoxy]pentanoic acid obtained in EXAMPLE 23 and 0.22 g of triethyl amine. The solution was cooled to -20 to -15°C while stirring and 0.3 g of isobutyl chloroformate was dropwise added thereto. After stirring for 30 minutes at the same temperature, 0.6 g of conc. ammonia water (ammonia 29%) was dropwise added to the mixture. After completion of the dropwise addition, the temperature was elevated to room temperature and stirring was continued for further 15 minutes.

Ice water was poured into the reaction solution and the organic layer was fractionated. The organic layer was successively washed with a saturated aqueous hydrogen sodium carbonate solution and water and then dried over anhydrous magnesium sulfate. Then the solvent was removed under reduced pressure and the resulting residue was subjected to silica gel column chromatography. The product was eluted out using chloroform-methanol (50:1 in a volume ratio) as an eluant. After the solvent was removed in vaccuo, the formed crystals were recrystallized from a solvent mixture of ether-benzene to give the product 2,2-dimethyl-5-[p-(2-methyl-4-thiazolyl)phenoxy]pentamide, as white crystals.
Melting point: 148 - 150°C

Elemental Analysis (as $C_{17}H_{22}N_2O_2S$)

| | C(%) | H(%) | (N%) | (S%) |
|---|---|---|---|---|
| Calcd. | 64.12 | 6.96 | 8.80 | 10.07 |
| Found | 64.20 | 7.04 | 8.78 | 9.95 |

## Claims

1. A phenoxy compound represented by general formula (I), or a salt thereof

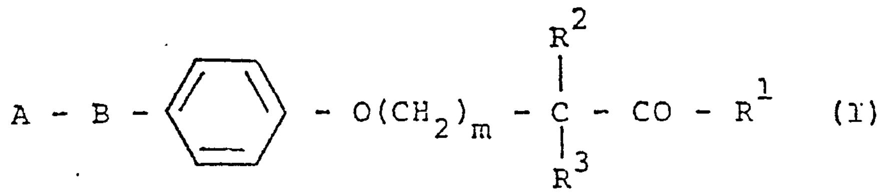

wherein:A is an imidazolyl group, a pyridyl group, a $C_1$-$C_5$ alkyl group, an amino group which may be substituted with a phenyl group or a $C_1$-$C_5$ alkyl group, or a carbamoyl group substituted with a cyclohexylamino group or an imidazolylalkyl group;
B is a thiazolyl group which may be substituted with a $C_1$-$C_5$ alkyl group;
m is 0 or an integer of 1 to 6;
$R^1$is a hydroxyl group, an amino group or a $C_1$-$C_5$ alkoxy group; and
$R^2$ and $R^3$ are the same or different and selected from a hydrogen atom and $C_1$-$C_5$ alkyl groups.

2. A compound according to claim 1 wherein A is a $C_1$-$C_5$ alkyl group or a pyridyl group, B is a thiazolyl group, $R^1$ is a $C_1$-$C_5$ alkoxy group and $R^2$, $R^3$ and $R^4$ are $C_1$-$C_5$ alkyl groups.

3. A pharmaceutical composition comprising a phenoxy compound according to claim 1 or 2.

4. A process for preparing a phenoxy compound represented by the following formula, or a salt thereof

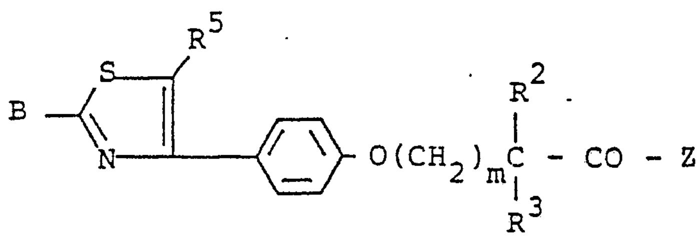

wherein
B is an imidazolyl group, a pyridyl group, a $C_1$-$C_5$ alkyl group, an amino group which may be substituted with a phenyl group or a $C_1$-$C_5$ alkyl group, or a carbamoyl group substituted with a cycloalkylamino group or an imidazolylalkyl group;
m is an integer of 1 to 6;
$R^5$is a hydrogen atom or a $C_1$-$C_5$ alkyl group;
$R^2$, $R^3$ are the same or different and selected from a hydrogen atom and $C_1$-$C_5$ alkyl groups; and
Z is an (optionally protected) hydroxy or $C_1$-$C_5$ alkoxy group or an amino group; which comprises reacting a thioamide derivative represented by general formula:

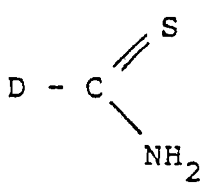

wherein D represents an imidazolyl group, a pyridyl group, a $C_1$-$C_5$ alkyl group,or an amino group which may be substituted with a phenyl group or a $C_1$-$C_5$ alkyl group, with a halide represented by general formula:

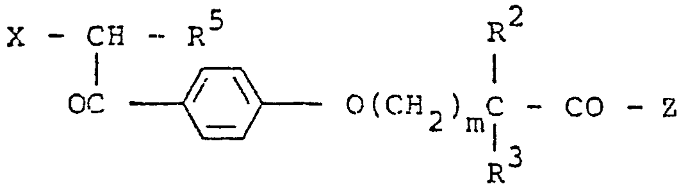

wherein X represents a halogen atom and, when D is a free amino group, optionally reacting with a cycloalkyl isocyanate or an imidazolylalkylenecarboxylic acid.

5. The product of any of Reference Examples 1 to 5 herein.